(19) Europäisches Patentamt
European Patent Office
Office européen des brevets

(11) EP 1 487 955 B1

(12) **EUROPÄISCHE PATENTSCHRIFT**

(45) Veröffentlichungstag und Bekanntmachung des Hinweises auf die Patenterteilung:
**21.05.2008 Patentblatt 2008/21**

(21) Anmeldenummer: **03714809.5**

(22) Anmeldetag: **12.03.2003**

(51) Int Cl.:
***C11D 3/39*** *(2006.01)* ***C07C 255/24*** *(2006.01)*

(86) Internationale Anmeldenummer:
**PCT/EP2003/002543**

(87) Internationale Veröffentlichungsnummer:
**WO 2003/078561 (25.09.2003 Gazette 2003/39)**

# (54) AMMONIUMNITRILE UND DEREN VERWENDUNG ALS HYDROPHOBE BLEICHAKTIVATOREN

AMMONIUM NITRILES AND THE USE THEREOF AS HYDROPHOBIC BLEACHING ACTIVATORS

NITRILES D'AMMONIUM ET LEUR UTILISATION COMME ACTIVATEURS DE BLANCHIMENT HYDROPHOBES

(84) Benannte Vertragsstaaten:
**BE CH DE ES FR GB IT LI**

(30) Priorität: **15.03.2002 DE 10211389**

(43) Veröffentlichungstag der Anmeldung:
**22.12.2004 Patentblatt 2004/52**

(73) Patentinhaber: **Clariant Produkte (Deutschland) GmbH**
**65929 Frankfurt am Main (DE)**

(72) Erfinder:
- **SEEBACH, Michael**
**65719 Hofheim (DE)**
- **REINHARDT, Gerd**
**65779 Kelkheim (DE)**

(56) Entgegenhaltungen:
**EP-A- 0 303 520** **EP-A- 0 464 880**
**US-B1- 6 174 853**

- **DAVID B. LUTEN, JR: "The Preparation of Aminonitriles and their Quaternary Ammonium Derivatives" JOURNAL OF ORGANIC CHEMISTRY, Nr. 3, 1938, Seiten 588-597, XP002245667**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Diese Erfindung betrifft Ammoniumnitrile sowie deren Verwendung zur Verstärkung der Bleichwirkung von Persauerstoffverbindungen beim Bleichen von gefärbten Anschmutzungen sowohl an Textilien wie auch an harten Oberflächen, sowie Wasch- und Reinigungsmittel, die diese Nitrile als Bleichaktivatoren enthalten.

Anorganische Persauerstoffverbindungen, insbesondere Wasserstoffperoxid und feste Persauerstoffverbindungen, die sich in Wasser unter Freisetzung von Wasserstoffperoxid lösen, wie Natriumperborat und Natriumcarbonat-Perhydrat, werden seit langem als Oxidationsmittel zu Desinfektions- und Bleichzwecken verwendet. Die Oxidationswirkung dieser Substanzen hängt in verdünnten Lösungen stark von der Temperatur ab; so erzielt man beispielsweise mit Wasserstoffperoxid oder Perborat in alkalischen Bleichflotten erst bei Temperaturen oberhalb von etwa 80°C eine ausreichend schnelle Bleiche verschmutzter Textilien.

Es ist bekannt, dass die Oxidationswirkung peroxidischer Bleichmittel, wie Perborate, Percarbonate, Persilikate und Perphosphate bei niedrigen Temperaturen verbessert werden kann, indem man Vorstufen von bleichenden Peroxysäuren, sogenannte Bleichaktivatoren, zusetzt. Viele Substanzen sind nach dem Stand der Technik als Bleichaktivatoren bekannt. Gewöhnlich handelt es sich dabei um reaktive organische Verbindungen mit einer O-Acyl- oder N-Acyl-Gruppe, die in alkalischer Lösung zusammen mit einer Quelle für Wasserstoffperoxid die entsprechenden Peroxysäuren bilden. Repräsentative Beispiele für Bleichaktivatoren sind etwa N,N,N',N'-Tetraacetylethylendiamin (TAED), Glucosepentaacetat (GPA), Xylosetetraacetat (TAX), Natrium-4-benzoyloxybenzolsulfonat (SBOBS), Natriumtrimethylhexanoyloxybenzolsulfonat (STHOBS), Tetraacetylglycoluril (TAGU), Tetraacetylcyansäure (TACA), Di-N-acetyldimethylglyoxim (ADMG), 1-Phenyl-3-acetylhydantoin (PAH), Natrium-nonanoyloxy-benzolsulfonat (NOBS) und Natrium-isononanoyloxy-benzolsulfonat (ISONOBS).

Durch Zusatz dieser Substanzen kann die Bleichwirkung wässriger Peroxid lösungen so weit gesteigert werden, dass bereits bei Temperaturen um 60°C im wesentlichen die gleichen Wirkungen wie mit der Peroxidlösung allein bei 95°C eintreten.

Mittlerweile haben einige kationische Verbindungen, die eine quartäre Ammoniumgruppe enthalten, an Bedeutung gewonnen, da sie hocheffektive Bleichaktivatoren darstellen. Solche kationischen Bleichaktivatoren sind beispielsweise in GB-A-1 382 594, US-A-4 751 015, EP-A-0 284 292, EP-A-0 331 229 beschrieben.

Ammoniumnitrile der Formel

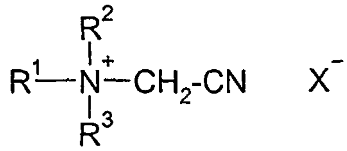

bilden dabei eine besondere Klasse kationischer Bleichaktivatoren. Verbindungen dieser Art und deren Verwendung als Aktivatoren in Bleichmitteln sind beschrieben in EP-A-303 520, EP-A-458 396 und EP-A-464 880. Bei den dort beschriebenen Verbindungen ist das Stickstoffatom der Ammoniumgruppe durch Alkyl-, Alkenyl- oder Arylgruppen substituiert, wobei höchstens einer der Substituenten eine größere Kettenlänge als C4 aufweist. Ammoniumnitrile dieses Typs, wobei zwei der Gruppen $R^1$, $R^2$ oder $R^3$ eine langkettige Alkylgruppe darstellen, fallen unter die allgemeine Formel in WO 98/23719 und WO 00/36061, sind aber dort nicht ausdrücklich vorbeschrieben.

Wahrscheinlich bilden diese Verbindungen bei der Perhydrolyse eine Peroxyimidsäure, welche als bleichendes Agens wirkt.

Die beschriebenen Verbindungen entfalten ihre Bleichwirkung vor allem an hydrophilen Anschmutzungen wie Tee oder Rotwein, während ihre Wirksamkeit an hydrophoben Anschmutzungen wie Curry- oder Ketchupflecken deutlich verringert ist.

Überraschenderweise wurde nun gefunden, dass Ammoniumnitrile der zuvor beschriebenen Art, die mindestens zwei Alkyl-, Alkenyl- oder Alkylether-Substituenten mit einer Kettenlänge größer C4 aufweisen, an hydrophoben Anschmutzungen eine bessere Bleichwirkung entfalten als die Nitrile gemäß dem Stand der Technik.

Gegenstand der vorliegenden Erfindung sind somit Verbindungen der allgemeinen Formel

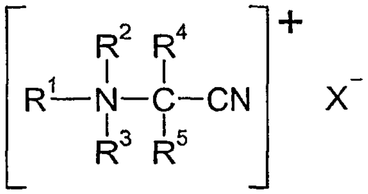

worin $R^1$ und $R^2$ jeweils einzeln eine gerad- oder verzweigtkettige $C_5$- bis $C_{24}$-Alkyl-, Alkenyl- oder Alkylethergruppe, vorzugsweise eine $C_5$- bis $C_{18}$-Alkyl-, Alkenyl- oder Alkylethergruppe bedeuten,
$R^3$ $C_1$- bis $C_{24}$-Alkyl, $C_2$- bis $C_{24}$-Alkenyl, Cyanomethyl oder $C_1$-$C_4$-Alkoxy-$C_1$- bis $C_4$-Alkyl, vorzugsweise $C_1$- bis $C_8$-Alkyl, $C_2$- bis $C_8$-Alkenyl oder $C_1$-Alkoxy-$C_1$- bis $C_4$-Alkyl ist,
$R^4$ und $R^5$ Wasserstoff, $C_1$- bis $C_4$-Alkyl, $C_2$- bis $C_4$-Alkenyl, $C_1$-$C_4$-Alkoxy-$C_1$-$C_4$-Alkyl, Phenyl, $C_1$- bis $C_3$-Alkylphenyl sind oder zusammen mit dem gemeinsamen Kohlenstoffatom eine $C_5$- bis $C_7$-Cycloalkylgruppe bilden, vorzugsweise Wasserstoff, Methyl oder Phenyl, wobei insbesondere $R_4$ Wasserstoff bedeutet, wenn $R_5$ kein Wasserstoff ist, und
$X^-$ ein Anion ist, beispielsweise Chlorid, Bromid, Jodid, Fluorid, Sulfat, Hydrogensulfat, Carbonat, Hydrogencarbonat, Phosphat, Mono- und Dihydrogenphosphat, Pyrophosphat, Metaphosphat, Nitrat, Methosulfat, Dodecylsulfat, Dodecylbenzolsulfonat, Phosphonat, Methylphosphonat, Methandisulfonat, Methylsulfonat, Ethansulfonat, Toluolsulfonat, Benzolsulfonat oder Cumolsulfonat, ausgenommen die Verbindung der Formel

$$(CH_3)(i\text{-}C_5H_{11})N^{\oplus}CH_2CN,\ J^-.$$

Besonders bevorzugt sind solche kationischen Nitrile, bei denen
$R^1$ und $R^2$ $C_6$- bis $C_{10}$-Alkyl bedeutet,
$R^3$ $C_1$- bis $C_6$-Alkyl ist,
$R^4$ und $R^5$ für Wasserstoff stehen und
$X^-$ Chlorid, Hydrogensulfat, Sulfat, Methosulfat, Toluolsulfonat, Benzolsulfonat oder Cumolsulfonat bedeutet.

Beispiele für diese besonders bevorzugten kationischen Nitrile sind (Cyanomethyl)-di-n-hexyl-methyl-ammonium-tosylat, (Cyanomethyl)-methyl-di-n-octyl-ammonium-chlorid, (Cyanomethyl)-ethyl-di-n-hexyl-ammonium-methosulfat, (Cyanomethyl)-di-n-decyl-methyl-ammonium-hydrogensulfat, (Cyanomethyl)-di-n-hexyl-methyl-ammonium-benzolsulfonat oder (Cyanomethyl)-di-n-octyl-methylammonium-cumolsulfonat.

Anhand einiger allgemeiner Beispiele sollen die Synthesewege für die kationischen Nitrile dieser Erfindung dargestellt werden:

1. Das sekundäre Amin der Formel $NHR^1R^2$ wird zusammen mit einer Base, vorzugsweise Alkalicarbonat oder Alkalihydroxid, in einem Lösemittel, vorzugsweise in absolutem Ethanol oder in einem Toluol/Wasser-Gemisch, vorgelegt. Bei Temperaturen zwischen 0 und 50°C, vorzugsweise bei 10 bis 30°C, wird Chloracetonitril zugetropft. Nach 1 bis 50 Stunden Reaktionszeit wird die organische Phase abgetrennt und die wässrige Phase mit einem organischen Lösungsmittel extrahiert. Von den vereinigten organischen Phasen wird das Lösungsmittel abgezogen. Das erhaltene Rohprodukt kann durch fraktionierende Destillation weiter gereinigt werden. Das entstandene Dialkylaminoacetonitril wird in einem organischen Lösungsmittel aufgenommen und mit einem Alkylierungsmittel wie Methylchlorid, Dimethylsulfat oder Arylsulfonsäurealkylester bei Temperaturen zwischen 20 und 100°C zum entsprechenden N-Cyanomethyl-ammonium-Salz umgesetzt. Das Salz kann durch herkömmliche Methoden der Aufarbeitung, wie Extraktion, Kristallisation, Abnutschen, Waschen des Kristallbreis auf der Nutsche und Trocknen, gewonnen werden.

2. Tertiäres Amin und Chloracetonitril werden in einem geeigneten Lösungsmittel, z.B. in Aceton für 1 bis 12 Stunden bei Temperaturen zwischen 10 und 70°C zur Reaktion gebracht. Der entstandene Niederschlag, das N-Cyanomethyl-ammonium-Chlorid wird abfiltriert, mit einem organischen Lösungsmittel gewaschen und getrocknet.

3. Sekundäres Amin, Natriumcyanid und ein Aldehyd oder ein Keton, vorzugsweise Formaldehyd in Form einer 36%igen Formalinlösung, werden in einem Lösungsmittel, vorzugsweise ein Ethanol/Wasser-Gemisch oder Wasser zusammengegeben. Nach einer Reaktionszeit von 1 bis 12 Stunden bei Temperaturen zwischen 10 und 80°C, vorzugsweise bei 10 bis 30°C wird dem Ansatz wässrige Salzsäure zugesetzt. Die wässrige Phase wird mit einem geeigneten organischen Lösungsmittel, z.B. Methylenchlorid oder Diethylether extrahiert. Von den vereinigten organischen Phasen wird nach Trocknen über Magnesiumsulfat das Lösungsmittel abgezogen. Das erhaltene Rohprodukt kann durch fraktionierende Destillation weiter gereinigt werden. Das entstandene Dialkylaminoacetonitril

wird in einem organischen Lösungsmittel aufgenommen und mit einem Alkylierungsmittel wie Methylchlorid, Dimethylsulfat oder Arylsulfonsäurealkylester bei Temperaturen zwischen 20 und 100°C zum entsprechenden N-Cyanomethyl-ammonium-Salz umgesetzt. Das Salz kann durch herkömmliche Methoden der Aufarbeitung, wie Extraktion, Kristallisation, Abnutschen, Waschen des Kristallbreis auf der Nutsche und Trocknen, gewonnen werden.

Gegenstand der Erfindung ist auch die Verwendung dieser Ammoniumnitrile als Bleichaktivatoren in bleichenden Wasch- und Reinigungsmitteln.
Unter dem Begriff der Bleiche wird hier sowohl das Bleichen von sich auf der Textiloberfläche befindendem Schmutz als auch das Bleichen von in der Waschflotte befindlichem, von der textilen Oberfläche abgelöstem Schmutz verstanden. Für das Bleichen von auf harten Oberflächen befindlichen Anschmutzungen gilt sinngemäß das gleiche. Weitere potentielle Anwendungen finden sich im Personal Care Bereich z.B. bei der Bleiche von Haaren und zur Verbesserung der Wirksamkeit von Gebissreinigern. Des weiteren finden die erfindungsgemäßen Komplexe Verwendung in gewerblichen Wäschereien, bei der Holz- und Papierbleiche, der Bleiche von Baumwolle und in Desinfektionsmitteln.
Weiterhin betrifft die Erfindung ein Verfahren zur Reinigung von Textilien wie auch von harten Oberflächen, insbesondere von Geschirr, unter Einsatz der genannten kationischen Nitrile in wässriger, gegebenenfalls weitere Wasch- beziehungsweise Reinigungsmittelbestandteile, insbesondere Oxidationsmittel auf Persauerstoffbasis, enthaltender Lösung, und Waschmittel sowie Reinigungsmittel für harte Oberflächen, insbesondere Reinigungsmittel für Geschirr, wobei solche für den Einsatz in maschinellen Verfahren bevorzugt sind, die derartige kationischen Nitrile enthalten.
Die erfindungsgemäße Verwendung besteht im wesentlichen darin, in Gegenwart einer mit gefärbten Anschmutzungen verunreinigten harten Oberfläche beziehungsweise eines entsprechend verschmutzten Textils Bedingungen zu schaffen, unter denen ein peroxidisches Oxidationsmittel und das kationische Nitril miteinander reagieren können, mit dem Ziel, stärker oxidierend wirkende Folgeprodukte zu erhalten. Solche Bedingungen liegen insbesondere dann vor, wenn die Reaktionspartner in wässriger Lösung aufeinander treffen. Dies kann durch separate Zugabe der Persauerstoffverbindung und des kationischen Nitrils zu einer gegebenenfalls Wasch- beziehungsweise Reinigungsmittel-haltigen Lösung geschehen. Besonders vorteilhaft wird das erfindungsgemäße Verfahren jedoch unter Verwendung eines erfindungsgemäßen Waschmittels beziehungsweise Reinigungsmittels für harte Oberflächen, das die kationischen Nitrile und gegebenenfalls ein persauerstoffhaltiges Oxidationsmittel enthält, durchgeführt. Die Persauerstoffverbindung kann auch separat in Substanz oder als vorzugsweise wässrige Lösung oder Suspension zur Lösung zugegeben werden, wenn ein persauerstofffreies Wasch- oder Reinigungsmittel verwendet wird.
Die erfindungsgemäßen Wasch- und Reinigungsmittel, die als Granulate, pulver- oder tablettenförmige Feststoffe, als sonstige Formkörper, homogene Lösungen oder Suspensionen vorliegen können, können außer dem genannten bleichverstärkenden Wirkstoff im Prinzip alle bekannten und in derartigen Mitteln üblichen Inhaltsstoffe enthalten. Die erfindungsgemäßen Mittel können insbesondere Buildersubstanzen, oberflächenaktive Tenside, Persauerstoffverbindungen, zusätzliche Persauerstoff-Aktivatoren oder organische Persäuren, wassermischbare organische Lösungsmittel, Sequestrierungsmittel, Verdickungsmittel, Konservierungsmittel, Perlglanzmittel, Emulgatoren und Enzyme, sowie spezielle Additive mit farb- oder faserschonender Wirkung enthalten. Weitere Hilfsstoffe wie Elektrolyte, pH-Regulatoren, Silberkorrosionsinhibitoren, Schaumregulatoren sowie Farb- und Duftstoffe sind möglich.
Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 bis 15 Gew.-%, enthalten.
Geeignete peroxidische Bleichmittel sind Wasserstoffperoxid und unter den Wasch- und Reinigungsbedingungen Wasserstoffperoxid abgebende Verbindungen wie Alkalimetallperoxide, organische Peroxide wie Harnstoff-Wasserstoffperoxid-Addukte und anorganische Persalze, wie Alkaliperborate, -percarbonate, -perphosphate, -persilikate, -persulfate und -peroxynitrite. Mischungen aus zwei oder mehreren dieser Verbindungen sind ebenfalls geeignet. Besonders bevorzugt sind Natriumperborat-Tetrahydrat und insbesondere Natriumperborat-Monohydrat sowie Natriumpercarbonat. Natriumperborat-Monohydrat ist wegen seiner guten Lagerbeständigkeit und seiner guten Löslichkeit in Wasser bevorzugt. Natriumpercarbonat kann aus ökologischen Gründen bevorzugt sein.
Alkalihydroperoxide sind eine weitere geeignete Gruppe von Peroxidverbindungen. Beispiele für diese Stoffe sind Cumolhydroperoxid und t-Butylhydroperoxid.
Auch aliphatische oder aromatische Mono- oder Dipercarbonsäuren sowie die entsprechenden Salze eignen sich als Peroxyverbindungen. Beispiele hierfür sind Peroxynaphthoesäure, Peroxylaurinsäure, Peroxystearinsäure, N, N-Phthaloylaminoperoxycapronsäure, 1,12-Diperoxydodecandisäure, 1,9-Diperoxyazelainsäure, Diperoxysebacinsäure, Diperoxyisophthalsäure, 2-Decyldiperoxybutan-1,4-disäure und 4,4'-Sulfonyl-bisperoxybenzoesäure.
In derartigen Wasch- und Reinigungsmitteln kann der erfindungsgemäße kationische, nitrilische Bleichaktivator mit einem Gewichtsanteil von etwa 0,1 bis 20 %, bevorzugt von 0,5 bis 10 %, insbesondere von 0,5 bis 5,0 % enthalten sein, zusammen mit einer Peroxyverbindung. Der Gewichtsanteil dieser Peroxyverbindung beträgt gewöhnlich von 2 bis 40 %, bevorzugt von 4 bis 30 %, insbesondere von 10 bis 25 %.

In den Wasch- und Reinigungsmitteln können neben den erfindungsgemäßen kationischen, nitrilischen Bleichaktivatoren noch andere geeignete Bleichaktivatoren in den üblichen Mengen (ca. 1 bis 10 Gew.-%) enthalten sein. Als Bleichaktivatoren geeignet sind organische Verbindungen mit einer O-Acyl- oder N-Acyl-Gruppe, insbesondere aus der Gruppe der aktivierten Carbonsäureester, insbesondere Natrium-nonanoyloxy-benzolsulfonat, Natrium-isononanoyloxy-benzolsulfonat, Natrium-4-benzoyloxy-benzolsulfonat, Natriumtrimethylhexanoyloxy-benzolsulfonat, Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran, Lactone, Acylale, Carbonsäureamide, Acyllactame, acylierte Harnstoffe und Oxamide, N-acylierte Hydantoine, beispielsweise 1-Phenyl-3-acetylhydantoin, Hydrazide, Triazole, Hydrotriazine, Urazole, Diketopiperazide, Sulfurylamide mehrfach acylierte Alkylendiamine beispielsweise N,N,N',N'-Tetraacetylethylendiamin, acylierte Triazinderivate, insbesondere 1,5-Diacetyl-2,4-dioxohexahydro-1,3,5-triazin, acylierte Glykolurile, insbesondere Tetraacetylglykoluril, N-Acylimide, insbesondere N-Nonaoylsuccinimid, und acylierte Zuckerderivate, insbesondere Pentaacetylglukose (PAG), Pentaacetylfructose, Tetraacetylxylose und Octaacetyllactose, sowie acetyliertes, gegebenenfalls N-alkyliertes Glucamin und Gluconolacton und/oder N-acylierte Lactame, beispielsweise N-Benzoylcaprolactam, aber auch quaternäre Nitrilverbindungen, beispielsweise quaternäre Trialkylammoniumnitrilsalze wie sie in EP-A-303 520, EP-A-458 396 und EP-A-464 880 beschrieben sind, insbesondere das Cyanomethyltrimethylammoniumsalz, aber auch heterocyclisch substituierte quaternäre Nitrilverbindungen, wie in EP-A-790 244 beschrieben.

Zusätzlich zu den oben aufgeführten konventionellen Bleichaktivatoren oder an deren Stelle können auch Sulfonimine, offenkettige oder cyclische quaternäre Iminiumverbindungen wie Dihydroisochinoliniumbetaine und/oder weitere bleichverstärkende Übergangsmetallsalze beziehungsweise ein- oder mehrkernige Übergangsmetallkomplexe mit acyclischen oder makrocyclischen Liganden, enthalten sein.

Die Wasch- und Reinigungsmittel können ein oder mehrere Tenside enthalten, wobei insbesondere anionische Tenside, nichtionische Tenside und deren Gemische, aber auch kationische, zwitterionische und amphotere Tenside in Frage kommen. Derartige Tenside sind in erfindungsgemäßen Waschmitteln in Mengenanteilen von vorzugsweise 1 bis 50 Gew.-%, insbesondere von 3 bis 30 Gew.-%, enthalten, wohingegen in Reinigungsmitteln für harte Oberflächen normalerweise geringere Anteile, das heißt Mengen bis zu 20 Gew.-%, insbesondere bis zu 10 Gew.-% und vorzugsweise im Bereich von 0,5 bis 5 Gew.-% enthalten sind. In Reinigungsmitteln für den Einsatz in maschinellen Geschirrspülverfahren werden normalerweise schaumarme Verbindungen eingesetzt.

Geeignete anionische Tenside sind insbesondere Seifen und solche, die Sulfat- oder Sulfonat-Gruppen enthalten. Als Tenside vom Sulfonat-Typ kommen vorzugsweise $C_9$-$C_{13}$-Alkylbenzolsulfonate, Olefinsulfonate, das heißt Gemische aus Alken- und Hydroxyalkansulfonaten sowie Disulfonaten, wie man sie beispielsweise aus Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus $C_{12}$-$C_{18}$-Alkanen beispielsweise durch Sulfochlorierung oder Sulfoxidation mit anschließender Hydrolyse beziehungsweise Neutralisation gewonnen werden. Geeignet sind auch die Ester von alpha-Sulfofettsäuren (Estersulfonate), zum Beispiel die alpha-sulfonierten Methylester der hydrierten Kokos-, Palmkern- oder Talgfettsäuren, die durch Sulfonierung der Methylester von Fettsäuren pflanzlichen und/oder tierischen Ursprungs mit 8 bis 20 C-Atomen im Fettsäuremolekül und nachfolgende Neutralisation zu wasserlöslichen Mono-Salzen hergestellt werden.

Weitere geeignete Aniontenside sind sulfierte Fettsäureglycerinester, welche Mono-, Di- und Triester sowie deren Gemische darstellen. Als Alk(en)ylsulfate werden die Alkali- und insbesondere die Natriumsalze der Schwefelsäurehalbester der $C_{12}$-$C_{18}$-Fettalkohole beispielsweise aus Kokosfettalkohol, Talgfettalkohol, Lauryl-, Myristyl-, Cetyl- oder Stearylalkohol oder der $C_8$-$C_{20}$-Oxoalkohole und diejenigen Halbester sekundärer Alkohole dieser Kettenlänge bevorzugt. Weiterhin bevorzugt sind Alk(en)ylsulfate der genannten Kettenlänge, welche einen synthetischen, auf petrochemischer Basis hergestellten geradkettigen Alkylrest enthalten. Auch 2,3-Alkylsulfate, sind geeignete Anionentenside. Geeignet sind auch die Schwefelsäurermonoester der mit 1 bis 6 Mol Ethylenoxid ethoxylierten geradkettigen oder verzweigten Alkohole, wie 2-Methylverzweigte $C_9$-$C_{11}$-Alkohole mit im Durchschnitt 3,5 Mol Ethylenoxid (EO) oder $C_{12}$-$C_{18}$-Fettalkohole mit 1 bis 4 EO.

Zu den bevorzugten Aniontensiden gehören auch die Salze der Alkylsulfobernsteinsäure, die auch als Sulfosuccinate oder als Sulfobernsteinsäureester bezeichnet werden, und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten $C_8$-$C_{18}$-Fettalkoholreste oder Mischungen aus diesen. Als weitere anionische Tenside kommen Fettsäurederivate von Aminosäuren, beispielsweise von N-Methyltaurin (Tauride) und/oder von N-Methylglycin (Sarkosinate) in Betracht. Als weitere anionische Tenside kommen insbesondere Seifen, beispielsweise in Mengen von 0,2 bis 5 Gew.-%, in Betracht. Geeignet sind insbesondere gesättigte Fettsäureseifen, wie die Salze der Laurinsäure, Myristinsäure, Palmitinsäure, Stearinsäure, hydrierten Erucasäure und Behensäure sowie insbesondere aus natürlichen Fettsäuren, z.B. Kokos-, Palmkern- oder Talgfettsäuren, abgeleitete Seifengemische.

Die anionischen Tenside, einschließlich der Seifen, können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor. Anionische

Tenside sind in erfindungsgemäßen Waschmitteln vorzugsweise in Mengen von 0,5 bis 10 Gew.-% und insbesondere in Mengen von 5 bis 25 Gew.-% enthalten.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm, Talgfett- oder Oleylalkohol, und durchschnittlich 2 bis 8 EO pro Mol Alkohol bevorzugt. Zu den bevorzugten ethoxylierten Alkoholen gehören beispielsweise $C_{12}$-$C_{14}$-Alkohole mit 3 EO oder 4 EO, $C_9$-$C_{11}$-Alkohole mit 7 EO, $C_{13}$-$C_{15}$-Alkohole mit 3 EO, 5 EO, 7 EO oder 8 EO, $C_{12}$-$C_{18}$-Alkohole mit 3 EO, 5 EO oder 7 EO und Mischungen aus diesen, wie Mischungen aus $C_{12}$-$C_{14}$-Alkohol mit 3 EO und $C_{12}$-$C_{18}$-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind (Talg-) Fettalkohole mit 14 EO, 16 EO, 20 EO, 25 EO, 30 EO oder 40 EO.

Zu den nichtionischen Tensiden zählen auch Alkylglykoside der allgemeinen Formel $RO(G)_x$, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und 6 für eine Glykosideinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl - die als analytisch zu bestimmende Größe auch gebrochene Werte annehmen kann - zwischen 1 und 10; vorzugsweise liegt x bei 1,2 bis 1,4. Ebenfalls geeignet sind Polyhydroxyfettsäureamide der Formel (I), in der Rest $R^1CO$ für einen aliphatischen Acylrest mit 6 bis 22 Kohlenstoffatomen, $R^2$ für Wasserstoff; einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht

(I) 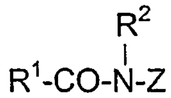

(II) 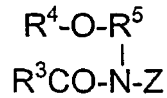

Vorzugsweise leiten sich die Polyhydroxyfettsäureamide von reduzierenden Zuckern mit 5 oder 6 Kohlenstoffatomen, insbesondere von der Glucose ab. Zur Gruppe der Polyhydroxyfettsäureamide gehören auch Verbindungen der Formel (II) $R^3$ für einen linearen oder verzweigten Alkyl- oder Alkenylrest mit 7 bis 12 Kohlenstoffatomen, $R^4$ für einen linearen, verzweigten oder cyclischen Alkylenrest oder einen Arylenrest mit 2 bis 8 Kohlenstoffatomen und $R^5$ für einen linearen, verzweigten oder cyclischen Alkylrest oder einen Arylrest oder einen Oxy-Alkylrest mit 1 bis 8 Kohlenstoffatomen steht, wobei $C_1$-$C_4$-Alkyl- oder Phenylreste bevorzugt sind, und [Z] für einen linearen Polyhydroxyalkylrest, dessen Alkylkette mit mindestens zwei Hydroxylgruppen substituiert ist, oder alkoxylierte, vorzugsweise ethoxylierte oder propoxylierte Derivate dieses Restes steht. [Z] wird auch hier vorzugsweise durch reduktive Aminierung eines Zuckers wie Glucose, Fructose, Maltose, Lactose, Galactose, Mannose oder Xylose erhalten. Die N-Alkoxy- oder N-Alyloxy-substituierten Verbindungen können dann durch Umsetzung mit Fettsäuremethylestern in Gegenwart eines Alkoxids als Katalysator in die gewünschten Polyhydroxyfettsäureamide überführt werden.

Eine weitere Klasse bevorzugt eingesetzter nichtionischer Tenside, die entweder als alleiniges nichtionisches Tensid oder in Kombination mit anderen nichtionischen Tensiden, insbesondere zusammen mit alkoxylierten Fettalkoholen und/oder Alkylglykosiden, eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Auch nichtionische Tenside vom Typ der Aminoxide, beispielsweise N-Kokosalkyl-N,N-dimethylaminoxid und N-Talgalkyl-N,N-dihydroxyethylaminoxid und der Fettsäurealkanolamide können geeignet sein.

Als weitere Tenside kommen sogenannte Gemini-Tenside in Betracht. Hierunter werden im allgemeinen solche Verbindungen verstanden, die zwei hydrophile Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, dass die hydrophilen Gruppen einen ausreichenden Abstand haben, damit sie unabhängig voneinander agieren können. Derartige Tenside zeichnen sich allgemeinen durch eine ungewöhnlich geringe kritische Micellkonzentration und die Fähigkeit, die Oberflächenspannung des Wassers stark zu reduzieren, aus. Eingesetzt werden können aber auch Gemini-Polyhydroxyfettsäureamide oder Poly-Polyhydroxyfettsäureamide. Weitere Tensidtypen können dendrimere Strukturen aufweisen.

Als organische und anorganische Gerüststoffe (Builder) eignen sich neutral oder insbesondere alkalisch reagierende Salze, die Calciumionen ausfällen oder komplexieren können. Geeignet und insbesondere ökologisch unbedenkliche Buildersubstanzen, sind kristalline, schichtförmige Silikate der allgemeinen Formel $NaMSi_{(x)}O_{(2x+1)}$, wobei M für Natrium oder Wasserstoff, x für eine Zahl von 1,9 bis 22, vorzugsweise von 1,9 bis 4 und y für eine Zahl von 0 bis 33 steht, beispielsweise Na-SKS-5 ($\alpha$-$Na_2Si_2O_5$), Na-SKS-7 ($\beta$-$Na_2Si_2O_5$, Natrosilit), Na-SKS-9 ($NaHSi_2O_5$*$H_2O$), Na-SKS-10 ($NaHSi_2O_3$*$3H_2O$, Kanemit), Na-SKS-11 (t-$Na_2Si_2O_5$) und Na-SKS-13 ($NaHSi_2O_5$), insbesondere aber Na-SKS-6 ($\delta$-$Na_2Si_2O_5$) sowie feinkristalline, synthetische wasserhaltige Zeolithe, insbesondere vom Typ NaA, die ein Calciumbindevermögen im Bereich von 100 bis 200 mg CaO/g aufweisen.
Zeolithe und Schichtsilikate können in einer Menge bis zu 20 Gew.-% im Mittel enthalten sein.
Des weiteren eignen sich nicht oder teilweise neutralisierte (co)polymere Polycarbonsäuren. Hierzu gehören die Homopolymere der Acrylsäure oder der Methacrylsäure bzw. deren Copolymere mit weiteren ethylenisch ungesättigten Monomeren wie beispielsweise Acrolein, Dimethylacrylsäure, Ethylacrylsäure, Vinylessigsäure, Allylessigsäure, Maleinsäure, Fumarsäure, Itaconsäure, Meth(-allylsulfonsäure), Vinylsulfonsäure, Styrolsulfonsäure, Acrylamidomethylpropansulfonsäure sowie Phosphorgruppen enthaltende Monomere wie beispielsweise Vinylphosphorsäure, Allylphosphorsäure und Acrylamidomethylpropanphosphorsäure und deren Salze, sowie Hydroxyethyl(meth)acrylatsulfat, Allylalkoholsulfat und Allylalkoholphosphate.

Bevorzugte (Co-)Polymere weisen eine mittlere Molmasse von 1000 bis 100 000 g/mol, vorzugsweise von 2000 bis 75000 g/mol und insbesondere von 2000 bis 35000 g/mol auf.
Der Neutralisierungsgrad der Säuregruppen liegt vorteilhafterweise bei 0 bis 90 %, vorzugsweise bei 10 bis 80 % und insbesondere bei 30 bis 70 %.

Zu den geeigneten Polymeren zählen vor allem auch Homopolymere der Acrylsäure und Copolymere der (Meth-)Acrylsäure mit Maleinsäure bzw. Maleinsäureanhydrid.

Weitere geeignete Copolymere leiten sich von Terpolymeren ab, die sich durch Polymerisation von 10 bis 70 Gew.-% monoethylenisch ungesättigten Dicarbonsäuren mit 4 bis 8 C-Atomen, deren Salzen, 20 bis 85 Gew.-% monoethylenisch ungesättigten Monocarbonsäuren mit 3 bis 10 C-Atomen bzw. deren Salzen, 1 bis 50 Gew.-% einfach ungesättigten Monomeren, welche nach der Verseifung Hydroxylgruppen an der Polymerkette freisetzen, und 0 bis 10 Gew.-% weiteren, radikalisch copolymerisierbaren Monomeren erhalten lassen.

Ebenfalls geeignet sind Pfropfpolymerisate von Monosacchariden, Oligosacchariden, Polysacchariden und modifizierten Polysacchariden sowie tierischen oder pflanzlichen Proteinen.
Bevorzugt sind Copolymerisate aus Zucker und anderen Polyhydroxyverbindungen und einer Monomermischung aus 45 bis 96 Gew.-% monoethylenisch ungesättigten $C_3$- bis $C_{10}$-Monocarbonsäuren oder Mischungen von $C_3$- bis $C_{10}$-Monocarbonsäuren und/oder deren Salze mit einwertigen Kationen, 4 bis 55 Gew.-% monoethylenisch ungesättigte Monosulfonsäuregruppen enthaltende Monomere, monoethylenisch ungesättigte Schwefelsäureester, Vinylphosphorsäureester und/oder die Salze dieser Säuren mit einwertigen Kationen sowie 0 bis 30 Gew.-% wasserlösliche ungesättigte Verbindungen, die mit 2 bis 50 Mol Alkylenoxid pro Mol monoethylenisch ungesättigter Verbindungen modifiziert sind.

Weitere geeignete Polymere sind Polyasparaginsäure bzw. deren Derivate in nicht oder nur teilneutralisierter Form.
Besonders geeignet sind auch Pfropfpolymerisate von Acrylsäure, Methacrylsäure, Maleinsäure und weiteren ethylenisch ungesättigten Monomeren auf Salze der Polyasparaginsäure, wie sie üblicherweise bei der zuvor beschriebenen Hydrolyse des Polysuccinimids anfallen. Hierbei kann auf die sonst notwendige Zugabe von Säure zur Herstellung der nur teilweise neutralisierten Form der Polyasparaginsäure verzichtet werden. Die Menge an Polyaspartat wird üblicherweise so gewählt, dass der Neutralisationsgrad aller im Polymerisat eingebauten Carboxylgruppen 80 %, vorzugsweise 60 %, nicht überschreitet.

Weitere einsetzbare Gerüststoffe sind beispielsweise die bevorzugt in Form ihrer Natriumsalze eingesetzten Percarbonsäuren, wie Zitronensäure, insbesondere Trinatriumcitrat und Trinatriumcitratdihydrat, Nitrilotriessigsäure und ihre wasserlöslichen Salze; die Alkalimetallsalze der Carboxymethyloxybernsteinsäure, Ethylendiamintetraessigsäure, Mono-, Dihydroxybernsteinsäure, a-Hydroxypropionsäure, Gluconsäure, Mellithsäure, Benzopolycarbonsäuren und solche wie in US-P-4 144 226 und 4 146 495 offenbart.
Auch phosphathaltige Builder, beispielsweise Alkaliphosphate, die in Form ihrer alkalischen neutralen oder sauren Natrium- oder Kaliumsalze vorliegen können, sind geeignet.
Beispiele hierfür sind Trinatriumphosphat, Tetranatriumdiphosphat, Dinatriumdihydrogenphosphat, Pentanatriumtriphosphat, sogenanntes Natriumhexametaphosphat, oligomeres Trinatriumphosphat mit Oligomerisierungsmengen im Bereich von 5 bis 1000, insbesondere 5 bis 50, sowie Gemische aus Natrium- und Kaliumsalzen.
Diese Buildersubstanzen können von 5 bis 80 Gew.-% enthalten sein, bevorzugt ist ein Anteil von 10 bis 60 Gew.-%.

Die gewünschte Viskosität der flüssigen Mittel kann durch Zugabe von Wasser und/oder organischen Lösungsmitteln oder durch Zugabe einer Kombination aus organischen Lösungsmitteln und Verdickungsmitteln eingestellt werden.

Prinzipiell kommen als organische Lösungsmittel alle ein- oder mehrwertigen Alkohole in Betracht. Bevorzugt werden Alkohole mit 1 bis 4 Kohlenstoffatomen, wie Methanol, Ethanol, Propanol, Isopropanol, geradkettiges und verzweigtes Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Eine vorteilhafte Mischung aus Lösungsmitteln besteht aus monomerem Alkohol, beispielsweise Ethanol und Polyethylenglykol im Verhältnis 0,5 : 1 bis 1,2 : 1.

Weitere geeignete Lösungsmittel sind beispielsweise Triacetin (Glycerintriacetat) und 1-Methoxy-2-propanol.

Als Verdickungsmittel werden bevorzugt gehärtetes Rizinusöl, Salze von langkettigen Fettsäuren, die vorzugsweise in Mengen von 0 bis 5 Gew.-% und insbesondere in Mengen von 0,5 bis 2 Gew.-%, beispielsweise Natrium-, Kalium-, Aluminium-, Magnesium- und Titanstearate oder die Natrium- und/oder Kaliumsalze der Behensäure, sowie Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid eingesetzt.
Als Verdicker eignen sich wasserlösliche Polyacrylate, die beispielsweise mit etwa 1 % eines Polyallylethers der Sucrose quervernetzt sind und die eine relative Molekülmasse oberhalb einer Million besitzen. Beispiele hierfür sind die unter dem Namen Carbopol® 940 und 941 erhältlichen Polymere. Die quervernetzten Polyacrylate werden in Mengen nicht über 1 Gew.-%, vorzugsweise in Mengen von 0,2 bis 0,7 Gew.-% eingesetzt.

Zu den in erfindungsgemäßen Mitteln gegebenenfalls enthaltenen Enzymen gehören Proteasen, Amylasen, Pullulanasen, Cellulasen, Cutinasen und/oder Lipasen, beispielsweise Proteasen wie BLAP®, Optimase®, Opticiean®, Maxacal®, Maxapem®, Durazym®, Purafect® OxP, Esperase® und/oder Savinase®, Amylasen wie Termamy®, Amylase-LT, Maxamyl®, Duramyl®, Purafectel OxAm, Cellulasen wie Celluzyme®, Carezyme®, K-AC® und/oder die aus den internationalen Patentanmeldungen WO 96/34108 und WO 96/34092 bekannten Cellulasen und/oder Lipasen wie Lipolase®, Lipomax®, Lumafast® und/oder Lipozym®. Die verwendeten Enzyme können, wie zum Beispiel in den internationalen Patentanmeldungen WO 92/111347 oder WO 94/23005 beschrieben, an Trägerstoffen adsorbiert und/oder in Hüllsubstanzen eingebettet sein, um sie gegen vorzeitige Inaktivierung zu schützen. Sie sind in erfindungsgemäßen Wasch- und Reinigungsmitteln vorzugsweise in Mengen bis zu 10 Gew.-%, insbesondere von 0,05 bis 5 Gew.-%, enthalten, wobei besonders bevorzugt gegen oxidativen Abbau stabilisierte Enzyme, eingesetzt werden.

Vorzugsweise enthalten erfindungsgemäße maschinelle Geschirrreinigungsmittel die üblichen Alkaliträger wie zum Beispiel Alkalisilikate, Alkalicarbonate und/oder Alkalihydrogencarbonate. Zu den üblicherweise eingesetzten Alkaliträgern zählen Carbonate, Hydrogencarbonate und Alkalisilikate mit einem Molverhältnis $SiO_2/M_20$ (M = Alkaliatom) von 1 : 1 bis 2,5 : 1. Alkalisilikate können dabei in Mengen von bis zu 40 Gew.-%, insbesondere 3 bis 30 Gew.-%, bezogen auf das gesamte Mittel, enthalten sein. Das in erfindungsgemäßen Reinigungsmitteln bevorzugt eingesetzte Alkaliträgersystein ist ein Gemisch aus Carbonat und Hydrogencarbonat, vorzugsweise Natriumcarbonat und -hydrogencarbonat, das in einer Menge von bis zu 50 Gew.-%, vorzugsweise 5 bis 40 Gew.-%, enthalten sein kann.

Ein weiterer Erfindungsgegenstand ist ein Mittel zum maschinellen Reinigen von Geschirr, enthaltend 15 bis 65 Gew.-%, insbesondere 20 bis 60 Gew.-% wasserlösliche Builderkompenente, 5 bis 25 Gew.-%, insbesondere 8 bis 17 Gew.-%. Bleichmittel auf Sauerstoffbasis, jeweils; bezogen auf das gesamte Mittel, und 0,1 bis 5 Gew.-% einer oder mehrerer der oben definierten kationischen nitrilischen Aktivatoren. Ein derartiges Mittel ist vorzugsweise niederalkalisch, das heißt seine Gewichtsprozentige Lösung weist einen pH-Wert von 8 bis 11,5, insbesondere 9 bis 11 auf.

In einer weiteren Ausführungsform erfindungsgemäßer Mittel zur automatischen Reinigung von Geschirr sind 20 bis 60 Gew.-% wasserlöslicher organischer Builder, insbesondere Alkalicitrat, 3 bis 20 Gew.-% Alkalicarbonat und 3 bis 40 Gew.-% Alkalidisilikat enthalten.

Um einen Silberkorrosionsschutz zu bewirken, können in erfindungsgemäßen Reinigungsmitteln für Geschirr Silberkorrosionsinhibitoren eingesetzt werden. Bevorzugte Silberkorrosionsschutzmittel sind organische Sulfide wie Cystin und Cystein, zwei- oder dreiwertige Phenole, gegebenenfalls alkyl- oder arylsubstituierte Triazole wie Benzotriazol, Isocyanursäure, Titan-, Zirkonium-, Hafnium-, Molybdän-, Vanadium- oder Cersalze und/oder -komplexe.

Sofern die Mittel bei der Anwendung zu stark schäumen, können ihnen noch bis zu 6 Gew.-%, vorzugsweise etwa 0,5 bis 4 Gew.-% einer schaumregulierenden Verbindung, vorzugsweise aus der Gruppe umfassend Silikone, Paraffine, Paraffin-Alkohol-Kombinationen, hydrophobierte Kieselsäuren, Bisfettsäureamide sowie deren Gemische und sonstige weitere bekannte im Handel erhältliche Schauminhibitoren zugesetzt werden. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt. Weitere fakultative Inhaltsstoffe in den erfindungsgemäßen Mitteln sind zum Beispiel Parfümöle.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel

sind in den erfindungsgemäßen Reinigungsmitteln vorzugsweise nicht über 20 Gew.-%, insbesondere von 1 bis 15 Gew.-%, vorhanden.

Zur Einstellung eines gewünschten, sich durch die Mischung der übrigen Komponenten nicht von selbst ergebenden pH-Werts können die erfindungsgemäßen Mittel System- und umweltverträgliche Säuren, insbesondere Zitronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, enthalten. Derartige pH-Regulatoren sind in den erfindungsgemäßen Mitteln vorzugsweise nicht über 10 Gew.-%, insbesondere von 0,5 bis 6 Gew.-%, enthalten.

Als Konservierungsmittel eignen sich beispielsweise Phenoxyethanol, Formaldehydlösung, Pentandiol oder Sorbinsäure.

Als Perlglanzmittel kommen beispielsweise Glycoldistearinsäureester wie Ethylenglycoldistearat, aber auch Fettsäuremonoglykolester in Betracht. Als Salze bzw. Stellmittel kommen beispielsweise Natriumsulfat, Natriumcarbonat oder Natriumsilikat (Wasserglas) in Betracht.

Als typische Einzelbeispiele für weitere Zusatzstoffe sind Natriumborat, Stärke, Saccharose, Polydextrose, RAED, Stilbenverbindungen, Methylcellulose, Toluolsulfonat, Cumolsulfonat, Seifen und Silicone zu nennen.

Die erfindungsgemäßen Mittel liegen vorzugsweise als pulverförmige, granulare oder tablettenförmige Präparate vor, die in an sich bekannter Weise, beispielsweise durch Mischen, Granulieren, Walzenkompaktieren und/oder durch Sprühtrocknung der thermisch belastbaren Komponenten und Zumischen der empfindlicheren Komponenten, zu denen insbesondere Enzyme, Bleichmittel und der Bleichkatalysator zu rechnen sind, hergestellt werden können. Erfindungsgemäße Mittel in Form wässriger oder sonstige übliche Lösungsmittel enthaltender Lösungen werden besonders vorteilhaft durch einfaches Mischen der Inhaltsstoffe, die in Substanz oder als Lösung in einen automatischen Mischer gegeben werden können, hergestellt.

Zur Herstellung von teilchenförmigen Mitteln mit erhöhtem Schüttgewicht, insbesondere im Bereich von 650 g/l bis 950 g/l, ist ein aus der europäischen Patentschrift EP 0 486 592 bekanntes, einen Extrusionsschritt aufweisendes Verfahren bevorzugt. Eine weitere bevorzugte Herstellung mit Hilfe eines Granulationsverfahrens ist in der europäischen Patentschrift EP 0 642 576 beschrieben. Die Herstellung erfindungsgemäßer Mittel in Form von nicht staubenden, lagerstabil rieselfähigen Pulvern und/oder Granulaten mit hohen Schüttdichten im Bereich von 800 bis 1000 g/l kann auch dadurch erfolgen, dass man in einer ersten Verfahrensstufe die Builder-Komponenten mit wenigstens einem Anteil flüssiger Mischungskomponenten unter Erhöhung der Schüttdichte dieses Vorgemisches vermischt und nachfolgend - gewünschtenfalls nach einer Zwischentrocknung - die weiteren Bestandteile des Mittels, darunter den kationischen, nitrilischen Aktivator, mit dem so gewonnenen Vorgemisch vereinigt.

Zur Herstellung von erfindungsgemäßen Mitteln in Tablettenform geht man vorzugsweise derart vor, dass man alle Bestandteile in einem Mischer miteinander vermischt und das Gemisch mittels herkömmlicher Tablettenpressen, beispielsweise Exzeriterpressen oder Rundläuferpressen verpresst. Man erhält so problemlos bruchfeste und dennoch unter Anwendungsbedingungen ausreichend schnell lösliche Tabletten mit Biegefestigkeiten von normalerweise über 150 N. Vorzugsweise weist eine derart hergestellte Tablette ein Gewicht von 1-5 g bis 40 g, insbesondere von 20 g bis 30 g auf; bei einem Durchmesser von 3-5 mm bis 40 mm.

Neben den bereits erwähnten Inhaltsstoffen können die Wasch- und Reinigungsmittel jeden der konventionellen Zusatzstoffe in Mengen enthalten, die man üblicherweise in solchen Mitteln vorfindet.

## Beispiele

### Beispiel 1: Synthese von (Cyanomethyl)-di-n-hexyl-methyl-ammoniumchlorid

10 g (0,05 mol) Dihexyl-methylamin wurden in 50 ml Aceton bei Raumtemperatur vorgelegt. Innerhalb von 10 Minuten tropfte man 3,8 g (0,05 mol) Chloracetonitril zu und rührte ca. 8 Stunden bei 50°C. Nach dieser Zeit zeigt die DC-Kontrolle kein Ausgangsprodukt mehr an. Der Ansatz wurde vollständig eingeengt und der Rückstand dreimal mit Diethylether gewaschen. Die vereinigten etherischen Phasen wurden am Rotationsverdampfer vollständig vom Lösungsmittel befreit. Man erhielt 12,7 g (0,046 mol) (Cyanomethyl)-di-n-hexyl-methylammoniumchlorid als gelbes, viskoses Öl, entsprechend einer Ausbeute von 92 %.

### Beispiel 2: Synthese von Di-n-hexylaminoacetonitril

95,6 g (0,5 mol) Di-n-hexylamin wurden in 200 ml absolutem Ethanol gelöst und mit 26,5 g (0,25 mol) Natriumcarbonat versetzt. Bei Raumtemperatur wurden innerhalb von 30 min 37,8 g (0,5 mol) Chloracetonitril zugetropft. Man rührte 16 Stunden bei 60°C nach und goss das Reaktionsgemisch auf 800 ml Wasser. Nach der Phasentrennung wurde die organische Phase dreimal mit jeweils 50 ml Wasser gewaschen, in Methylenchlorid aufgenommen, über Magnesiumsulfat getrocknet, filtriert und eingeengt. Man erhielt eine hellbraune Flüssigkeit, die durch fraktionierende

Destillation gereinigt wird. Die Fraktionen zwischen 104 bis111 °C bei 25 mbar wurden gesammelt. Man erhielt 95,6 g (0,43 mol) reines Din-Hexylamino-acetonitril, entsprechend einer Ausbeute von 86 %.

Beispiel 3: Synthese von (Cyanomethyl)-di-n-hexyl-methyl-ammonium-methosulfat

11,2 g (0,05 mol) Di-n-hexylaminoacetonitril wurden in 50 ml Acetonitril vorgelegt. Innerhalb von 5 Minuten tropfte man 6,3 g (0,05 mol) Dimethylsulfat zu und rührte den Ansatz ca. 120 Stunden bei Raumtemperatur. Die klare Lösung wurde am Rotationsverdampfer eingedampft und der Rückstand aus Diethylether auskristallisiert. Nach dem Absaugen des Kristallbreis wurde mit Diethylether nachgewaschen und im Vakuumtrockenschrank getrocknet. Man erhielt 16,8 g (0,048 mol) (Cyanomethyl)-di-n-hexyl-methylammonium-methosulfat als weißen kristallinen Feststoff, entsprechend einer Ausbeute von 96 %.

Beispiel 4: Synthese von (Cyanomethyl)-di-n-hexyl-methyl-ammonium-benzolsulfonat

18,0 g (0,08 mol) Dihexylaminoacetonitril wurden in 50 ml Essigsäureethylester vorgelegt. Zu dieser Lösung tropfte man bei Raumtemperatur innerhalb von 10 min 13,8 g (0,08 mol) Benzolsulfonsäuremethylester. Man rührte 24 Stunden bei Raumtemperatur nach und engte die klare Lösung am Rotationsverdampfer ein. Der Rückstand wurde mit Diethylether ausgerührt und der ausgefallene Feststoff abgenutscht. Nach dem Waschen mit Diethylether wurde bei 50°C im Vakuumtrockenschrank getrocknet. Man erhielt 20,5 g (0,052 mol) (Cyanomethyl)-di-n-hexyl-methylammonium-benzolsulfonat als weißes Pulver, entsprechend einer Ausbeute von 65 %.

Beispiel 5: Synthese von Di-n-octylaminoacetonitril

73,9 g (0,3 mol) Di-n-octylamin wurden zusammen mit 31,8 g (0,3 mol) Natriumcarbonat in 240 ml absolutem Ethanol vorgelegt. Bei Raumtemperatur gab man 22,9 g (0,3 mol) Chloracetonitril hinzu und rührte so lange bei Rückfluss nach, bis mittels Dünnschichtchromatographie (stationäre Phase: Kieselgel; mobile Phase: Methanol) kein Edukt mehr nachgewiesen werden konnte. Die Reaktionsmischung wurde filtriert und das Filtrat am Rotationsverdampfer zur Trokkene eingeengt. Der Rückstand wurde einer fraktionierenden Destillation unterworfen. Bei 145°C und 0,04 mbar erhielt man eine Fraktion, die nach NMR-Spektroskopie rein ist. Man isolierte 58,4 g (0,21 mol) Di-n-octylamino-acetonitril in Form einer klaren Flüssigkeit, entsprechend einer Ausbeute von 69 %.

Beispiel 6: Synthese von (Cyanomethyl)-di-n-octyl-methyl-ammonium-toluolsulfonat

28,1 g (0,1 mol) Di-n-octylaminoacetonitril wurden zusammen mit 18,6 g (0,1 mol) Toluolsulfonsäuremethylester in 100 ml Essigsäurethylester 16 Stunden bei 60°C gerührt. Die klare Lösung engte man am Rotationsverdampfer ein und rührte den Rückstand in Diethylether aus. Der erhaltene Feststoff wurde abgenutscht, mit Diethylether gewaschen und im Vakuum getrocknet. Man erhielt 15,7 g (0,034 mol) (Cyanomethyl)-di-n-octyl-methylammonium-toluolsulfonat als weißen Feststoff, entsprechend einer Ausbeute von 34 %.

Beispiel 7: Synthese von Cumolsulfonsäuremethylester

Man legte 594,9 g (5,0 mol) Thionylchlorid zusammen mit 1 g DMF vor und gab beginnend bei Raumtemperatur langsam 222,2 g (1,0 mol) Natriumcumolsulfonat portionsweise zu. Man rührte 16 Stunden am Rückfluss und gab nach dem Erkalten 250 ml Chloroform zu. Nach dem Filtrieren wurde überschüssiges Thionylchlorid zusammen mit dem Chloroform am Vakuum abgezogen. Man fügt 250 ml Methanol zu und stellte mit 25 %iger Natronlauge bei 25°C einen pH-Wert von 7,0 ein. Anschließend rührte man mit Dichlormethan aus, engte die organische Phase am Rotationsverdampfer ein und destillierte den erhaltenen Rückstand fraktionierend am Vakuum. Bei 102°C und 0,03 mbar isolierte man 96,9 g (0,44 mol) Cumolsulfonsäuremethylester in Form einer klaren Flüssigkeit, entsprechend einer Ausbeute von 44 %.

Beispiel 8: Synthese von (Cyanomethyl)-di-n-octyl-methylammonium-cumolsulfonat

28,1 g(0,1 mol) Di-n-octylaminoacetonitril wurden in 100 ml Essigsäureethylester vorgelegt und nach der Zugabe von 21,4 g (0,1 mol) Cumolsulfonsäuremethylester 16 Stunden lang am Rückfluss gerührt. Der erhaltene Feststoff wurde abgenutscht, mit Diethylether gewaschen und am Vakuum getrocknet. Man erhielt 20,0 g (0,04 mol) (Cyanomethyl)-di-n-octyl-methylammonium-cumolsulfonat in Form eines weißen Feststoffs, entsprechend einer Ausbeute von 40 %.

Beispiel 9:

Mit den erfindungsgemäßen kationischen Nitrilverbindungen wurden Bleichmittelformulierungen hergestellt, mit denen Waschversuche an einer hydrophoben Anschmutzung (Curry) durchgeführt wurden. Basis der Bleichmittelformulierungen war eine wässrige Lösung eines phosphatfreien Grundwaschmittels (Referenzwaschmittel WMP der WFK-Testgewebe GmbH Krefeld) mit einer Konzentration von 2 g/l WMP in Wasser von 15°dH. Hierzu wurden 0,5 g/l Natriumperborat-Monohydrat sowie unterschiedliche Mengen der kationischen Nitrilverbindungen eingewogen.

Mit diesen Formulierungen wurden bleichempfindliche Standardtestgewebe der Wäschereiforschung Krefeld (WFK) mit der Anschmutzung Curry (BC-4) in einem Linitest-Gerät (Heraeus) einer Behandlung bei einer Temperatur von 20 oder 40°C unter isothermen Waschbedingungen unterworfen. Nach einer dreißigminütigen Waschzeit wurden die Gewebestücke mit Wasser gespült, getrocknet und gebügelt. Anschließend wurde die Bleichwirkung durch eine Bestimmung der Differenz ΔR(Formulierung+Aktivator) der Remissionen vor und nach dem Waschvorgang mittels eines Weißgrad-Messgerätes (ELREPHO 2000, Datacolor) quantifiziert. Aus diesen ΔR(Formulierung+Aktivator)-Werten und den in Kontrollversuchen ohne kationischer Nitrilverbindung ermittelten Werten ΔR(Formulierung) wurden die in nachfolgenden Tabellen aufgelisteten ΔΔR-Werte berechnet, die ein direktes Maß für die durch den Zusatz an kationischer Nitrilverbindung hervorgerufene Verbesserung der Bleichwirkung darstellen:

$$\Delta\Delta R = \Delta R(\text{Formulierung+Aktivator}) - \Delta R(\text{Formulierung})$$

Es wurden Bleichmittelzusammensetzungen mit den erfindungsgemäßen kationischen Nitrilverbindungen 3 bis 7 sowie den Vergleichssubstanzen 1 und 2 hergestellt.

Die Verbindungen 1 bis 7 sind

1 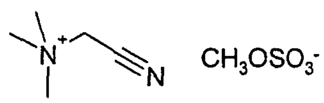

2 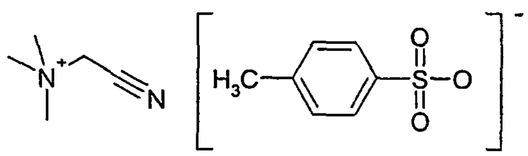

3 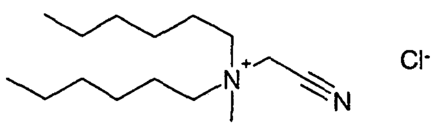

4 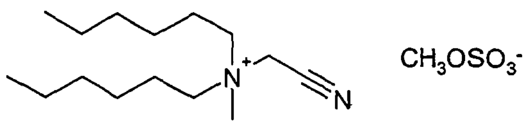

5 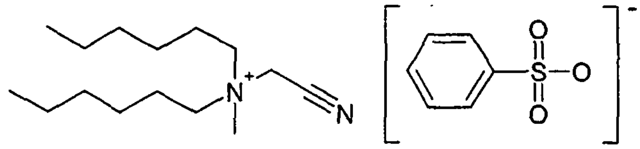

6 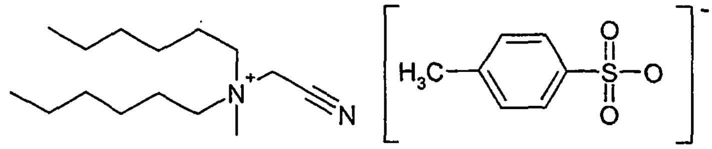

7 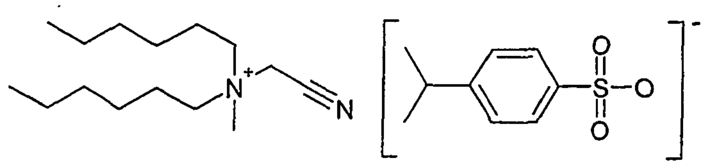

Mit den Aktivatoren 3 und 4 wurden Waschversuche bei 20 bzw. 40°C bei Konzentrationen von 0,3 bzw. 0,5 g/l durchgeführt. Die Ergebnisse werden in Tabelle 1 mit dem Aktivator 1 verglichen:

Tabelle 1: Prüfergebnisse (ΔΔR-Werte) für die Aktivatoren 3 und 4

| Waschbedingungen | Aktivator 3 | Aktivator 4 | Aktivator 1 |
|---|---|---|---|
| 20°C; c(Aktivator) = 0,3 g/l | 4,1 | 2,3 | 1,8 |
| 20°C; c(Aktivator) = 0,5 g/l | 4,0 | 3,5 | 2,0 |
| 40°C; c(Aktivator) = 0,3 g/l | 5,0 | 4,7 | 4,2 |
| 40°C; c(Aktivator) = 0,5 g/l | 7,3 | 5,6 | 4,1 |

Für die Aktivatoren 5 bis 7 wurden Waschversuche bei 20 bzw. 40°C bei einer Konzentration von 0,25 g/l durchgeführt. Die Ergebnisse werden in Tabelle 2 mit dem Aktivator 2 sowie mit TAED verglichen:

Tabelle 2: Prüfergebnisse (ΔΔR-Werte) für die Aktivatoren 5 bis 7; c(Aktivator) = 0,25 g/l

| Waschbedingungen | Aktivator 5 | Aktivator 6 | Aktivator 7 | Aktivator 2 | TAED |
|---|---|---|---|---|---|
| 20°C | 2,9 | 2,8 | 4,1 | 2,6 | 0,1 |
| 40°C | 4,6 | 4,6 | 5,6 | 3,1 | 1,3 |

Die Untersuchungen zeigen, dass die erfindungsgemäßen kationischen Nitrile an hydrophoben Anschmutzungen eine bessere Bleichwirkung entfalten als die kationischen Aktivatoren des Standes der Technik oder als TAED. Weitere nützliche Eigenschaften der kationischen Nitrile sind geringe Farbschädigung und geringe Faserschädigung.

## Patentansprüche

**1.** Verbindungen der allgemeinen Formel (I)

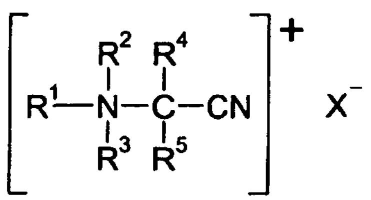 (I)

worin $R^1$ und $R^2$ jeweils einzeln eine gerad- oder verzweigtkettige $C_5$- bis $C_{24}$-Alkyl-, Alkenyl- oder Alkylethergruppe bedeuten,
$R^3$ $C_1$-$C_{24}$-Alkyl, $C_2$-$C_{24}$-Alkenyl, Cyanomethyl oder $C_1$-$C_4$-Alkoxy-$C_1$-$C_4$-Alkyl ist, $R^4$ und $R^5$ Wasserstoff, $C_1$-$C_4$-Alkyl, $C_2$-$C_4$-Alkenyl, $C_1$-$C_4$-Alkoxy-$C_1$-$C_4$-Alkyl, Phenyl, $C_1$- bis $C_3$-Alkylphenyl sind oder zusammen mit dem gemeinsamen Kohlenstoffatom eine $C_5$-$C_7$-Cycloalkylgruppe bilden, $R_4$ Wasserstoff bedeutet, wenn $R_5$ kein Wasserstoff ist, und
$X^-$ ein Anion ist, ausgenommen die Verbindung der Formel

$$(CH_3)(i\text{-}C_5H_{11})N^{\oplus}CH_2CN\ J^-.$$

2. Verbindungen der allgemeinen Formel (I) nach Anspruch 1, **dadurch gekennzeichnet, dass** $R^1$ und $R^2$ jeweils einzeln eine $C_5$-$C_{18}$-Alkyl-, Alkenyl- oder Alkylethergruppe bedeuten.

3. Verbindungen der allgemeinen Formel (I) nach Anspruch 1, **dadurch gekennzeichnet, dass** $R^3$ $C_1$-$C_8$-Alkyl, $C_2$-$C_8$-Alkenyl oder $C_1$-Alkoxy-$C_1$-$C_4$-Alkyl ist.

4. Verbindungen der allgemeinen Formel (I) nach Anspruch 1, **dadurch gekennzeichnet, dass** $R^4$ und $R^5$ Wasserstoff bedeuten.

5. Verbindungen der allgemeinen Formel (I) nach Anspruch 1, **dadurch gekennzeichnet, dass** $X^-$ ein Anion ist, beispielsweise Chlorid, Bromid, Jodid, Fluorid, Sulfat, Hydrogensulfat, Carbonat, Hydrogencarbonat, Phosphat, Mono- und Dihydrogenphosphat, Pyrophosphat, Metaphosphat, Nitrat, Methosulfat, Dodecylsulfat, Dodecylbenzolsulfonat, Phosphonat, Methylphosphonat, Methandisulfonat, Methylsulfonat, Ethansulfonat, Toluolsulfonat, Benzolsulfonat oder Cumolsulfonat ist.

6. Verbindungen der allgemeinen Formel (I) nach Anspruch 1, **dadurch gekennzeichnet, dass** $R^1$ und $R^2$ $C_6$-$C_{10}$-Alkyl bedeutet, $R^3$ $C_1$-$C_6$-Alkyl ist, $R^4$ und $R^5$ für Wasserstoff stehen und $X^-$ ein Anion bedeutet.

7. Verbindung der allgemeinen Formel (I) nach Anspruch 1, **dadurch gekennzeichnet, dass** $R^1$ und $R^2$ $C_6$-$C_{10}$-Alkyl bedeutet, $R^3$ $C_1$-$C_2$-Alkyl ist, $R^4$ und $R^5$ für Wasserstoff stehen und $X^-$ Chlorid, Hydrogensulfat, Sulfat, Methosulfat,-Toluolsulfonat, Benzolsulfonat oder Cumolsulfonat bedeutet.

8. Wasch-, Reinigungs- und Desinfektionsmittel enthaltend eine Verbindung der Formel (I) gemäß Anspruch 1.

9. Maschinengeschirrspülmittel enthaltend eine Verbindung der Formel (I) gemäß Anspruch 1.

10. Waschmittelformulierungen enthaltend eine Verbindung der Formel (I) gemäß Anspruch 1.

11. Verwendung der Verbindungen nach Anspruch 1 bei der Bleiche von Textilmaterial und Papier.

## Claims

1. A compound of the formula (I)

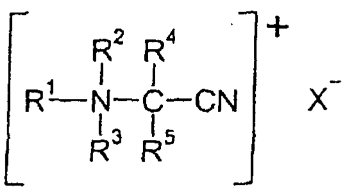

(I)

in which $R^1$ and $R^2$ are in each case individually a straight-chain or branched-chain $C_5$-$C_{24}$-alkyl, alkenyl or alkyl ether group,
$R^3$ is $C_1$-$C_{24}$-alkyl, $C_2$-$C_{24}$-alkenyl, cyanomethyl or $C_1$-$C_4$-alkoxy-$C_1$-$C_4$-alkyl ,
$R^4$ and $R^5$ are hydrogen, $C_1$-$C_4$-alkyl, $C_2$-$C_4$-alkenyl, $C_1$-$C_4$-alkoxy-$C_1$-$C_4$-alkyl, phenyl, $C_1$- to $C_3$-alkylphenyl or together with the common carbon atom form a $C_5$-$C_7$-cycloalkyl group, $R_4$ is hydrogen if $R_5$ is not hydrogen, and

$X^-$ is an anion, excluding the compound of the formula $(CH_3)$ $(i\text{-}C_5H_{11})N^+CH_2CHI^-$ .

2. A compound of the formula (I) as claimed in claim 1, wherein $R^1$ and $R^2$ are in each case individually a $C_5$-$C_{18}$-alkyl, alkenyl or alkyl ether group.

3. A compound of the formula (I) as claimed in claim 1, wherein $R^3$ is $C_1$-$C_8$-alkyl, $C_2$-$C_8$-alkenyl or $C_1$-alkoxy-$C_1$-$C_4$-alkyl.

4. A compound of the formula (I) as claimed in claim 1, wherein $R^4$ and $R^5$ are hydrogen.

5. A compound of the formula (I) as claimed in claim 1, wherein $X^-$ is an anion, for example chloride, bromide, iodide, fluoride, sulfate, hydrogensulfate, carbonate, hydrogencarbonate, phosphate, mono- and dihydrogenphosphate, pyrophosphate, metaphosphate, nitrate, methosulfate, dodecylsulfate, dodecylbenzenesulfonate, phosphonate, methylphosphonate, methanedisulfonate, methylsulfonate, ethanesulfonate, toluenesulfonate, benzenesulfonate or cumenesulfonate.

6. A compound of the formula (I) as claimed in claim 1, wherein $R^1$ and $R^2$ is $C_6$-$C_{10}$-alkyl, $R^3$ is $C_1$-$C_6$-alkyl, $R^4$ and $R^5$ are hydrogen and $X^-$ is an anion.

7. A compound of the formula (I) as claimed in claim 1, wherein $R^1$ and $R^2$ is $C_6$-$C_{10}$-alkyl, $R^3$ is $C_1$-$C_2$-alkyl, $R^4$ and $R^5$ are hydrogen and $X^-$ is chloride, hydrogensulfate, sulfate, methosulfate, toluenesulfonate, benzenesulfonate or cumenesulfonate.

8. A washing, cleaning or disinfecting composition comprising a compound of the formula (I) as claimed in claim 1.

9. A machine dishwashing composition comprising a compound of the formula (I) as claimed in claim 1.

10. A washing composition formulation comprising a compound of the formula (I) as claimed in claim 1.

11. The use of the compounds as claimed in claim 1 for the bleaching of textile material and paper.

**Revendications**

1. Composés de formule générale (I)

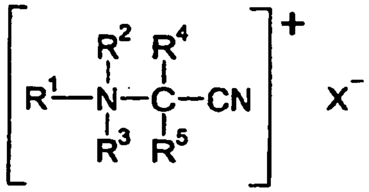

(I)

dans laquelle
$R^1$ et $R^2$ représentent, chacun indépendamment l'un de l'autre, un groupe alkyle en $C_5$ à $C_{24}$ linéaire ou ramifié, alcényle ou éther d'alkyle,
$R^3$ représente un groupe alkyle en $C_1$ à $C_{24}$, alcényle en $C_2$ à $C_{24}$, cyanométhyle ou (alcoxyle en $C_1$ à $C_4$)-(alkyle en $C_1$ à $C_4$),
$R^4$ et $R^5$ représentent un atome d'hydrogène, un groupe alkyle en $C_1$ à $C_4$, alcényle en $C_2$ à $C_4$, (alcoxyle en $C_1$ à $C_4$)-(alkyle en $C_1$ à $C_4$), phényle, (alkyle en $C_1$ à $C_3$)-phényle ou, conjointement avec l'atome de carbone commun, un groupe cycloalkyle en $C_5$ à $C_7$,
$R^4$ représente un atome d'hydrogène, si $R^5$ ne représente pas un atome d'hydrogène, et
$X^-$ est un anion, à l'exception du composé de formule $(CH_3)(i\text{-}C_5H_{11})N^+CH_2CNJ^-$.

2. Composés de formule générale (I) selon la revendication 1, **caractérisés en ce que** $R^1$ et $R^2$ représentent, chacun indépendamment l'un de l'autre, un groupe alkyle en $C_5$ à $C_{18}$, alcényle ou éther d'alkyle.

3. Composés de formule générale (I) selon la revendication 1, **caractérisés en ce que** $R^3$ représente un groupe alkyle en $C_1$ à $C_8$, alcényle en $C_2$ à $C_8$ ou (alcoxyle en $C_1$)-(alkyle en $C_1$ à $C_4$).

4. Composés de formule générale (I) selon la revendication 1, **caractérisés en ce que** $R^4$ et $R^5$ représentent un atome d'hydrogène.

5. Composés de formule générale (I) selon la revendication 1, **caractérisés en ce que** $X^-$ est un anion, par exemple le chlorure, le bromure, l'iodure, le fluorure, le sulfate, l'hydrogénosulfate, le carbonate, l'hydrogénocarbonate, le phosphate, le mono- et le di-hydrogénophosphate, le pyrophosphate, le métaphosphate, le nitrate, le méthosulfate, le dodécylsulfate, le dodécylbenzènesulfonate, le phosphonate, le méthylphosphonate, le méthanedisulfonate, le méthylsulfonate, l'éthanesulfonate, le toluènesulfonate, le benzènesulfonate ou le cumènesulfonate.

6. Composés de formule générale (I) selon la revendication 1, **caractérisés en ce que** $R^1$ et $R^2$ représentent un groupe alkyle en $C_6$ à $C_{10}$, $R^3$ représente un groupe alkyle en $C_1$ à $C_6$, $R^4$ et $R^5$ représentent un atome d'hydrogène et $X^-$ est un anion.

7. Composé de formule générale (I) selon la revendication 1, **caractérisé en ce que** $R^1$ et $R^2$ représentent un groupe alkyle en $C_6$ à $C_{10}$, $R^3$ représente un groupe alkyle en $C_1$ à $C_2$, $R^4$ et $R^5$ représentent un atome d'hydrogène et $X^-$ représente le chlorure, l'hydrogénosulfate, le sulfate, le méthosulfate, le toluènesulfonate, le benzènesulfonate ou le cumènesulfonate.

8. Produits de lavage, de nettoyage et de désinfection contenant un composé de formule (I) selon la revendication 1.

9. Produits pour vaisselle en machine contenant un composé de formule (I) selon la revendication 1.

10. Formulations de détergent contenant un composé de formule (I) selon la revendication 1.

11. Utilisation de composés selon la revendication 1 pour le blanchiment de matière textile et de papier.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- GB 1382594 A
- US 4751015 A
- EP 0284292 A
- EP 0331229 A
- EP 303520 A
- EP 458396 A
- EP 464880 A
- WO 9823719 A
- WO 0036061 A
- EP 790244 A
- US 4144226 P
- US 4146495 P
- WO 9634108 A
- WO 9634092 A
- WO 92111347 A
- WO 9423005 A
- EP 0486592 A
- EP 0642576 A